# EUROPEAN PATENT APPLICATION

(11) **EP 4 549 047 A1**
(43) Date of publication of application: **07.05.2025**
(21) Application number: 23831015.5
(22) Date of filing: 08.06.2023
(51) Int. Cl.: B21G 1/08, B21K 21/12, C21D 8/10, A61M 5/158, A61M 5/32

(54) **NON-MAGNETIC FINE STAINLESS STEEL PROCESSED PRODUCT AND METHOD FOR PRODUCING SAME**

(30) Priority: 28.06.2022 JP 2022103203; 13.07.2022 JP 2022112120
(71) Applicant: Terakata Manufacturing Co., Ltd,, Tottori 689-2103 (JP); Tottori Institute Of Industrial Technology, Tottori-shi, Tottori 689-1112 (JP)
(72) Inventor: TERAKATA, Yasuo, Tohaku-gun, Tottori 689-2103 (JP); TANAKA, Shinji, Tohaku-gun, Tottori 689-2103 (JP); IWATA, Masahiro, Tohaku-gun, Tottori 689-2103 (JP); YAMADA, Ryoichi, Tohaku-gun, Tottori 689-2103 (JP); MATSUDA,Tomoko, Yonago-shi, Tottori 689-3522 (JP); TERADA, Naofumi, Yonago-shi, Tottori 689-3522 (JP)
(74) Representative: Zacco GmbH
(86) International application number: PCT/JP2023/021311
(87) International publication number: WO 2024/004565

(57) **Abstract**

A non-magnetic fine stainless steel processed product having a tapered tip formed of a stainless crystal structure having a crystal grain size of 1 µm or less, at a tip end part of a body part formed of fine stainless steel, by a manufacture method including a tapered slope forming step of forming a tapered slope by cutting diagonally fine stainless steel, a tapered tip crush-forming step of forming a tapered tip by subjecting a tip end part of the tapered slope to warm crushing that is performed by keeping the tapered slope at a plastic deformation temperature of 200°C or more and 300°C or less with a temperature variation of 30°C or less, and a tapered tip trimming step of trimming the tapered tip.

## Description

### Technical Field

The invention of the present application relates to a non-magnetic fine stainless steel processed product and a method for manufacturing the same. In particular, the invention relates to a non-magnetic fine stainless steel processed product excellent in grindability, in which a stainless steel crystal grain size of a tapered tip is refined by warm crushing, and a method for manufacturing the same.

### Background Art

It is known that it is possible to provide a component in which generation of burrs is less likely to occur in association with secondary processing accuracy during punching and cutting, and a forming process, by refining the crystal grain of a metal material or an alloy material, and stainless steel having a fine grain structure is disclosed (Patent Literature 1).

However, the stainless steel having a fine structure is manufactured by cold-rolling and heat-treating a stainless steel sheet, and a fine stainless steel processed product in which the tip end part of the fine stainless steel is formed of a refined crystal structure is not disclosed.

An austenitic stainless steel material (SUS304) is excellent in corrosion resistance and mechanical properties and less expensive, and therefore is widely used in small-article precision components. Patent Literature 2 discloses a warm forging method for an austenitic stainless steel material (SUS304) that keeps a composition deformation temperature at 170°C or more and 400°C or less, with a temperature variation of 30°C or less by a temperature-controlled die and a thermally insulating mechanism. However, it does not suggest a non-magnetic fine stainless steel processed product excellent in grindability in which a refined crystal structure is formed by warm crushing at the tapered tip of the fine stainless steel and a method for manufacturing the same.

### Document List

### Patent Literatures

Patent Literature 1: Japanese Patent Application Publication No. 2020-084288
Patent Literature 2: WO 2018/143113

### Summary of Invention

### Technical Problem

It is an object of the invention of the present application to propose a non-magnetic fine stainless steel processed product in which generation of burrs due to a forming process is less likely to occur at a tapered tip, and a method for manufacturing the same.

### Solution to Problem

The problem to be solved by the invention of the present application can be solved by aspects (1) to (9) as follows. Specifically:
(Aspect 1) A non-magnetic fine stainless steel processed product is a non-magnetic fine stainless steel processed product having a tapered tip at an end part of a body part formed of fine stainless steel, and is characterized in that the tapered tip is formed of a stainless crystal structure having a crystal grain size of 1 µm or less.

By forming the tapered tip of a stainless crystal structure having a crystal grain size of 1 µm or less, it is possible to provide a non-magnetic fine stainless steel processed product in which generation of burrs due to a trimming process and a grinding process hardly occurs.

(Aspect 2) A non-magnetic fine stainless steel processed product that is a non-magnetic fine stainless steel processed product composed of a body part formed of fine stainless steel, a tapered slope circumferentially in contact with the body part and formed at a predetermined inclination angle θ with respect to an axis of the body part, and is characterized in that the inclination angle θ of the tapered slope is larger than the inclination angle ϕ of the tapered tip, and the tapered tip is formed of a stainless crystal structure having a crystal grain size of 1 µm or less.

By forming the tapered tip by crushing the tapered slope by warm crushing, the inclination angle of the tapered slope becomes larger than the inclination angle of the tapered tip. Further, by crushing the tapered slope, the stainless crystal structure having a crystal grain size of 1 µm or less is formed at the tapered tip, and it is possible to provide a non-magnetic fine stainless steel processed product in which generation of burrs due to a trimming process and a grinding process hardly occurs.

(Aspect 3) The non-magnetic fine stainless steel processed product according to either aspect 1 or aspect 2, characterized in that the fine stainless steel is either polygonal or cylindrical stainless steel, or polygonal or cylindrical hollow stainless steel, with a sectional area of 5 mm² or less.

In the fine stainless steel with the sectional area of 5 mm² or less, the tapered tip obtained by crushing the tapered slope by warm-crushing is easily formed of the stainless crystal structure having the crystal grain size of 1 µm or less.

(Aspect 4) The non-magnetic fine stainless steel processed product according to any one of aspect 1 to aspect 3, characterized in that the fine stainless steel is austenitic stainless steel.

Since austenitic stainless steel is generally used and is non-magnetic, it is used as medical surgical needles and medical scalpels.

(Aspect 5) A method for manufacturing a non-magnetic fine stainless steel processed product including a tapered slope forming step of forming a tapered slope by cutting diagonally fine stainless steel, a tapered tip crush-forming step of forming a tapered tip by warm-crushing a tip end part of the tapered slope, and a tapered tip trimming step of trimming the tapered tip that is warm-crushed.

By adopting the tapered tip crush-forming step of forming the tapered tip by warm-crushing the tip end part of the tapered slope, it is possible to make the crystal grain size of the crystal structure of the tapered tip 1 µm or less, and it is possible to provide the non-magnetic fine stainless steel processed product in which generation of burrs due to a trimming process and a griding process hardly occurs.

(Aspect 6) The method for manufacturing a non-magnetic fine stainless steel processed product according to aspect 5, characterized in that the fine stainless steel is either polygonal or cylindrical stainless steel, or polygonal or cylindrical hollow stainless steel, with a sectional area of 5 mm² or less.

In the fine stainless steel with the sectional area of 5 mm² or less, the tapered tip formed by crushing the tapered slope by warm crushing is easily formed of the stainless crystal structure having the crystal grain size of 1 µm or less.

(Aspect 7) The method for manufacturing a non-magnetic fine stainless steel processed product according to either aspect 5 or aspect 6, characterized in that the fine stainless steel is austenitic stainless steel.

Since austenitic stainless steel is generally used and is non-magnetic, it is used as medical surgical needles, and medical scalpels.

(Aspect 8) The method for manufacturing a non-magnetic fine stainless steel processed product according to any one of aspect 5 to aspect 7, characterized in that the tapered tip crush-forming step is warm crushing that is performed by keeping the tapered slope at a plastic deformation temperature of 200°C or more and 300°C or less with a temperature variation of 30°C or less.

By keeping the tapered slope at a plastic deformation temperature of 200°C or more and 300°C or less with a temperature variation of 30°C or less and warm-crushing the tapered slope, a process-induced martensitic transformation of the austenitic stainless steel material (SUS304) is prevented, and it is possible to provide a non-magnetic fine stainless steel processed product having a non-magnetic tapered tip excellent in corrosion resistance and mechanical properties.

(Aspect 9) The method for manufacturing a non-magnetic fine stainless steel processed product according to any one of aspect 5 to aspect 8, characterized in that the tapered tip is formed of a stainless crystal structure having a crystal grain size of 1 µm or less.

By forming the tapered tip of the stainless crystal structure having the crystal grain size of 1 µm or less, it is possible to provide a non-magnetic fine stainless steel processed product in which generation of burrs due to a trimming process and a grinding process hardly occurs.

### Effects of Invention

According to the invention of the present application, it is possible to provide a non-magnetic fine stainless steel processed product in which the stainless crystal structure of the tapered tip is formed with the crystal grain size of 1 µm or less and generation of burrs due to a trimming process and a grinding process hardly occurs. Further, by adopting the temperature-controlled warm crushing, a process-induced martensitic transformation of the tapered tip is prevented, and it is possible to provide a non-magnetic fine stainless steel processed product excellent in corrosion resistance, mechanical properties, and pointed end processibility, and non-magnetic.

### Brief Description of Drawings

[Fig. 1A] Fig. 1A is schematic views explaining one embodiment of a non-magnetic fine stainless steel processed product of the invention of the present application.
[Fig. 1B] Fig. 1B is schematic views explaining one embodiment of a non-magnetic fine stainless steel processed product of the invention of the present application.
[Fig. 1C] Fig. 1C is schematic views explaining one embodiment of a non-magnetic fine stainless steel processed product of the invention of the present application.
[Fig. 2] Fig. 2 is a crystal structure EBSD analysis image of a tapered tip of the non-magnetic fine stainless steel processed product of the invention of the present application.
[Fig. 3] Fig. 3 is a flowchart explaining a manufacturing flow of the non-magnetic fine stainless steel processed product of the invention of the present application.
[Fig. 4] Fig. 4 is schematic views illustrating modes of a tapered slope formed by wire electric discharge processing of the invention of the present application.
[Fig. 5A] Fig. 5A is photographs showing the tapered slope formed by wire electric discharge processing of the invention of the present application.
[Fig. 5B] Fig. 5B is photographs showing the tapered slope formed by wire electric discharge processing of the invention of the present application.
[Fig. 6A] Fig. 6A is photographs showing the non-magnetic fine stainless steel processed product obtained by performing warm-crushing by a press die of the invention of the present application.
[Fig. 6B] Fig. 6B is photographs showing the non-magnetic fine stainless steel processed product obtained by performing warm-crushing by a press die of the invention of the present application.
[Fig. 7A] Fig. 7A is photographs showing the tapered tip of the non-magnetic fine stainless steel processed product of the invention of the present application before and after trimming.
[Fig. 7B] Fig. 7B is photographs showing the tapered tip of the non-magnetic fine stainless steel processed product of the invention of the present application before and after trimming.
[Fig. 8A] Fig. 8A is photographs showing the trimmed tapered tip of the non-magnetic fine stainless steel processed product of the invention of the present application.
[Fig. 8B] Fig. 8B is photographs showing the trimmed tapered tip of the non-magnetic fine stainless steel processed product of the invention of the present application.

### Description of Embodiments

Hereinafter, an embodiment of a non-magnetic fine stainless steel processed product and a method for manufacturing the same of the invention of the present application will be described.

### (1) Non-magnetic Fine Stainless Steel Processed Product

### (1-1) Fine Stainless Steel

The non-magnetic fine stainless steel processed product of the invention of the present application is obtained by warm-crushing fine stainless steel.

The fine stainless steel of the invention of the present application is not particularly limited as long as it is polygonal or cylindrical stainless steel or polygonal or cylindrical hollow stainless steel, with a sectional area of 5 mm² or less. The fine stainless steel with a sectional area of 5 mm² or less is easy to form a crystal structure having a crystal grain size of 1 µm or less by warm crushing, and can be applied to medical instruments such as injection needles for treatments of eyes suffering from cataracts, glaucoma, and the like. For use in injection needles, cylindrical hollow stainless steel can be suitably used.

Further, as the fine stainless steel of the invention of the present application, austenitic stainless steel is suitable. Since it is highly versatile and non-magnetic, it is widely adopted for medical applications.

### (1-2) Fine Stainless Steel Processed Product

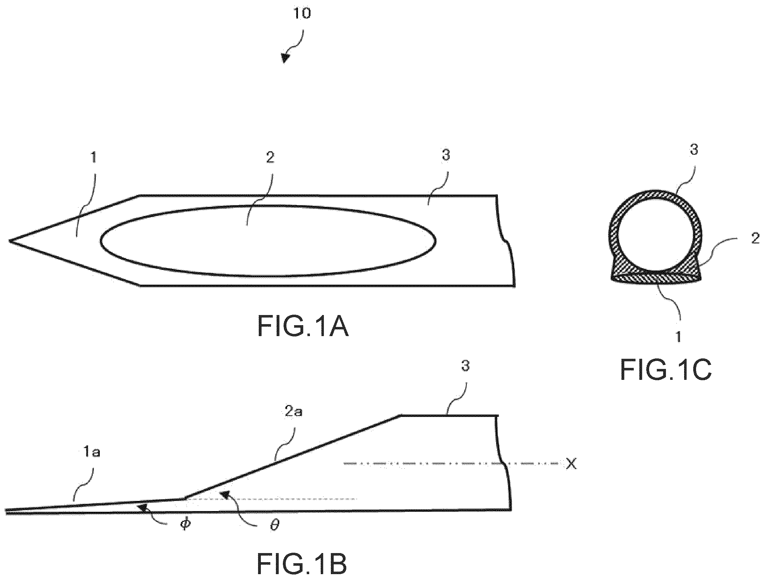

Fig. 1A, Fig. 1B, and Fig. 1C are schematic views explaining one embodiment of the fine stainless steel processed product of the invention of the present application, which are a plan view, a side view, and a front view respectively. A fine stainless steel processed product 10 of the invention of the present application is composed of a tapered incline part 2 formed by cutting diagonally a body part 3 made of cylindrical hollow stainless steel, and a tapered tip 1 obtained by warm-crushing the tapered slope 2.

The tapered slope 2 is circumferentially in contact with the body part 3 and forms an inclined surface 2a at an inclination angle θ with respect to an axis X of the body part 3. The tapered tip 1 is circumferentially in contact with the tapered slope 2 and forms an inclined surface 1a at an inclination angle ϕ with respect to the axis X of the body part 3. Since the tapered tip 1 is formed by warm-crushing the tapered slope 2, the inclination angle θ is larger than the inclination angle ϕ. Lengths of the inclined surfaces (1a, 2a) and the inclination angles (θ, ϕ) of the tapered slope 2 and the tapered tip 1 can be appropriately set by an angle at which the body part 3 is cut off, and a reduction rate of warm-crushing. The inclination angle θ of the tapered slope 2 is preferably 9° or more and 30° or less, and the inclination angle ϕ of the tapered tip 1 is preferably 2° or more to less than 9°.

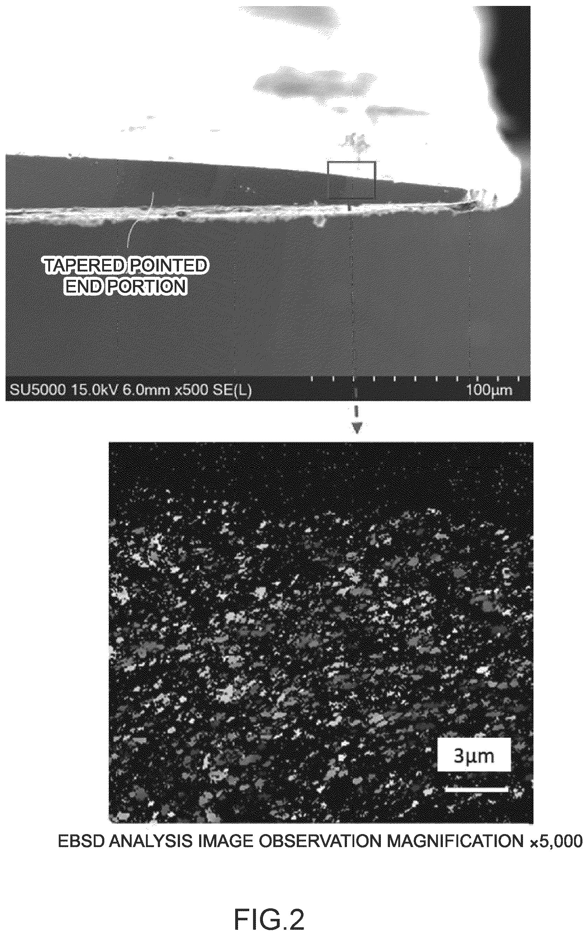

Fig. 2 is a crystal structure EBSD analysis image of the tapered tip 1 of the fine stainless steel processed product 10 of the invention of the present application. The tapered tip 1 is composed of a crystal structure having a crystal grain size of 1 µm or less. By refining the crystal grain size, generation of burrs due to a trimming process and a grinding process hardly occurs.

A relative magnetic permeability (µₛ) of the tapered tip 1 (reduction rate 65%) is 1.005, which is in a range of a relative magnetic permeability (µₛ) 1.003 to 1.007 inherent to austenitic stainless steel (SUS304), and is non-magnetic.

### (2) Manufacture Method

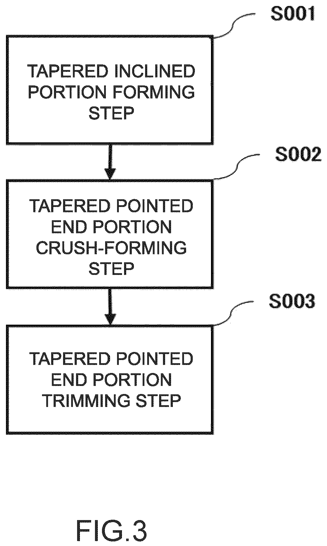

Fig. 3 is a flow chart explaining a manufacturing flow of the fine stainless steel processed product 10 of the invention of the present application. In the manufacture of the fine stainless steel processed product 10, a tapered slope forming step (S001), a tapered tip crush-forming step (S002), and a tapered tip trimming step (S003) are sequentially performed.

### (2-1) Tapered slope Forming Step

The tapered slope forming step (S001) is a step of tapering and cutting fine stainless steel held by a fixing jig (not shown) by wire electric discharge processing. The wire electric discharge processing is a processing method in which an electric current is passed through a wire (ultra-fine electrode wire) made of brass or the like to melt and cut a workpiece. Specifically, the fine stainless steel held by the fixing jig is installed in a processing tank filled with a processing liquid of a wire electric discharge processing device, and an electric current is passed through the wire to process it. By adopting the wire electric discharge processing, the taper shape of the tapered slope 2 can be processed with high accuracy, and various taper shapes can be made.

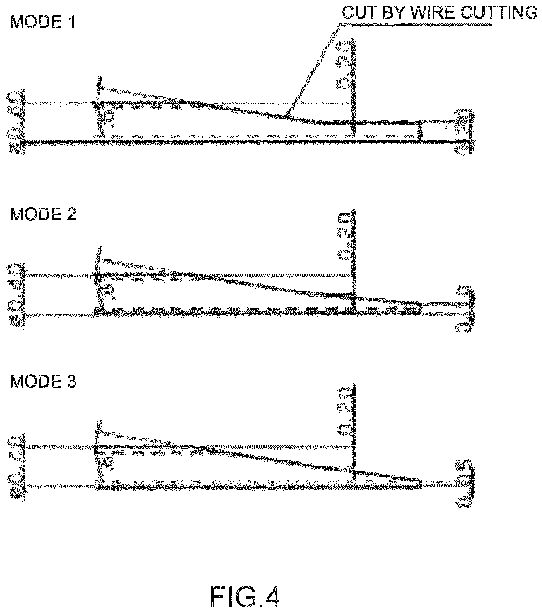

Fig. 4 is schematic views illustrating modes of the tapered slope 2 formed by wire electric discharge processing of the invention of the present application, and mode 1 to mode 3 respectively show modes in which thicknesses of the tip end parts are changed. By forming the tapered slopes 2 having different thicknesses of the tip end parts, it is possible to produce the fine stainless steel processed products 10 in which taper shapes of the tapered slopes 2 are the same, and the taper shapes of the tapered tips 1 are different.

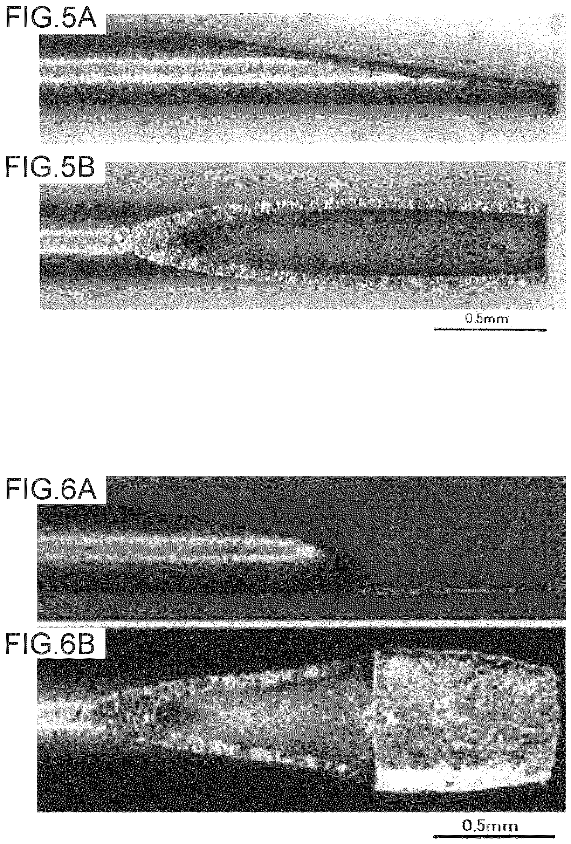

Fig. 5A and Fig. 5B are photographs showing the tapered slope 2 (mode 3) formed by wire electric discharge processing, which are a side surface photograph, and a plane photograph, respectively.

### (2-2) Tapered tip Crush-Forming Step

The tapered tip crush-forming step (S002) is a step of forming the tapered tip 1 by warm-crushing the tip end part of the tapered slope 2. Warm-crushing refers to a warm-forging method in which the tip end part of the tapered slope 2 is crushed using a temperature-controlled press die (not shown).

The reduction rate by crushing is preferably 50 to 70%. When the reduction rate is less than 50%, a crystal structure having a crystal grain size of 1 µm or less cannot be formed. When the reduction rate exceeds 70%, a life of the press die is shortened.

An excess thickness of the tip end part of the tapered slope 2, which is generated by warm rushing is trimmed by a punching die to form the tapered tip 1.

The temperature-controlled press die keeps the tip end part of the tapered slope at a temperature within a plastic deformation temperature range (200°C to 300°C) by a built-in heater. The press die is kept at a temperature in the plastic deformation temperature range of 200°C to 300°C with a temperature variation of 30°C or less by performing temperature measurement. The temperature measurement is performed by using a radiation thermometer that can measure at a high speed, and can measure in a non-contact manner. A temperature measurement value is captured in real time, and processed by a temperature control program, and the temperature is controlled by a die temperature control procedure.

Furthermore, by keeping the crushing temperature in the range of 200°C to 300°C, it is possible to keep the crushing temperature at a temperature exceeding a temperature (100°C) of generation of process-induced martensite and maintain non-magnetism of the austenitic stainless steel (SUS304). Accordingly, the tapered tip 1 can be made non-magnetic similarly to the body part 3 and the tapered slope 2, and the non-magnetic fine stainless steel processed product 10 can be realized.

The reduction rate of the warm crushing,
Fig. 6A and Fig. 6B are photographs showing the non-magnetic fine stainless steel processed product subjected to warm-crushing by the press die of the invention of the present application, which are a side surface photograph in which the tip end of mode 1 is warm-crushed, a plane photograph in which the tip end of mode 1 is warm-crushed, and a plane photograph in which a tip end of mode 3 is warm-crushed.

### (2-3) Tapered Tip Trimming Step

In the tapered tip trimming step (S003), an excess thickness of the tapered tip 1 generated by warm-crushing is trimmed by a punching die, and the tapered tip 1 is shaped.

### Example

An embodiment exhibiting the effect of the invention of the present application will be shown as an example.

### <Example 1>

### (1) Tapered slope Formation

The tapered slope was formed by wire electric discharge processing at the end part of a cylindrical hollow stainless steel (austenitic stainless steel (SUS304); φ0.4 mm, t0.05 mm). The inclination angle θ of the tapered slope was 9.40°.

### (2) Tapered Tip Crush-Forming

The tip end part of the tapered slope was warm-crushed by the press die controlled in temperature (200°C to 300°C) with the reduction rate of 65%. The inclination angle ϕ of the tapered tip was 7.85°.

Furthermore, the excess thickness of the tip end part of the tapered slope that is generated by warm-crushing was trimmed by a punching die, and the tapered tip was formed.

### (3) Tapered Tip Trimming

The excess thickness of the tapered tip 1 generated by warm-crushing was trimmed by the punching die, and the tapered tip 1 was shaped.

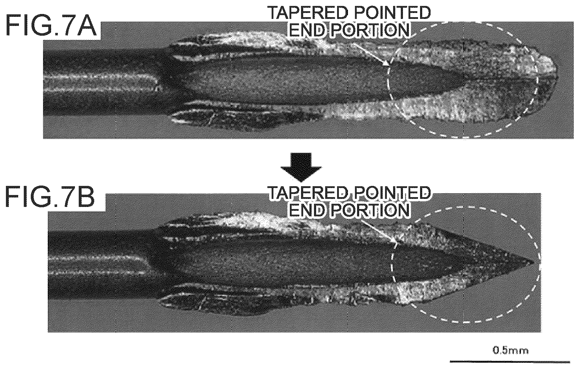

Fig. 7A and Fig.7B are plane photographs showing the fine stainless steel processed product produced in example 1, which are after warm-crushing, and after trimming respectively.

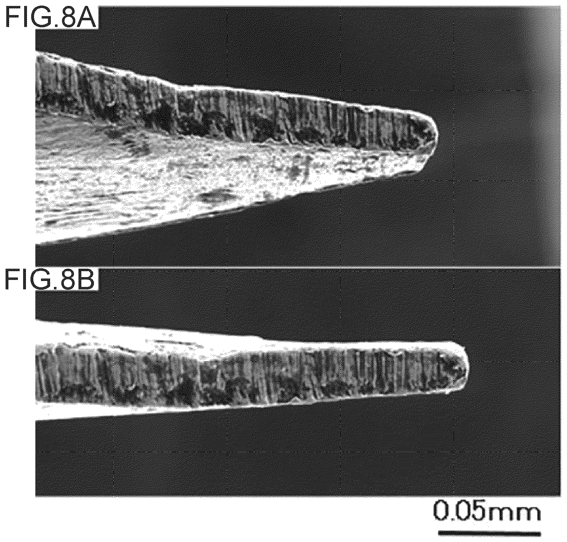

Fig. 8A and Fig. 8B are photographs showing the tapered tip that was trimmed, which are a rear perspective, and a side surface. Generation of burrs is not recognized.

### (4) Relative Magnetic Permeability Evaluation

Evaluation of the relative magnetic permeability (µₛ) was performed for the tapered tip of the fine stainless steel processed product in which the tapered tip was trimmed.

Here, the magnetic permeability is a proportional constant (µ) when relationship between an intensity (H) of a magnetic field and a magnetic flux density (B) is represented by B-µH. In addition, a ratio (µ/µ₀) to a vacuum magnetic permeability (µ₀) is referred to as the relative magnetic permeability (µₛ), and the relative magnetic permeability (µₛ) of homogeneous and isotropic medium is 1 in an optical wavelength region. It is 1.003 to 1.007 in the austenitic stainless steel (SUS304), and corresponds to non-magnetic stainless steel.

The relative magnetic permeability (µₛ) was measured by a magnetic property evaluation equipment (Magnetic Permeability Meter LP-141A made by Denshijiki Industry Co., Ltd.). The relative magnetic permeability (µₛ) of the tapered tip is 1.004, and maintains the original characteristics of the austenitic stainless steel (SUS304).

### (5) Crystal Structure Evaluation

The tapered tip of the fine stainless steel processed product in which the tapered tip was trimmed was subjected to crystal orientation analysis by EBDS, and an EBSD analysis image was obtained. Fig. 2 is the crystal tissue EBSD analysis image of the tapered tip of the fine stainless steel processed product, and the crystal grain size of the crystal structure was 1 µm or less.

### (6) Summary

In the fine stainless steel processed product having the tapered tip formed by the temperature-controlled warm-crushing of the invention of the present application, the relative magnetic permeability (µs) of the tapered tip is 1.004, and the body part, the tapered slope, and the tapered tip all maintain non-magnetism that is the characteristic of the austenitic stainless steel (SUS304). In addition, the crystal grain size of the tapered tip crystal structure is 1 µm or less, no burr is generated by trimming, and grindability was good.

### Industrial Applicability

According to the invention of the present application, it is possible to provide the non-magnetic fine stainless processed product having the tip end part excellent in grindability.

### List of Reference Signs

1 tapered tip,
1a tapered tip inclined surface,
2 tapered slope,
2a tapered slope inclined surface,
3 body part,
10 fine stainless steel processed product

## Claims

1. A non-magnetic fine stainless steel processed product having a tapered tip at an end part of a body part formed of stainless steel having a fine shape that is either polygonal or cylindrical stainless steel, or polygonal or cylindrical hollow stainless steel, with a sectional area of 5 mm² or less, **characterized in that** the tapered tip is formed of a stainless crystal structure having a crystal grain size of 1 µm or less.

2. A non-magnetic fine stainless steel processed product comprising a body part formed of stainless steel having a fine shape that is either polygonal or cylindrical stainless steel, or polygonal or cylindrical hollow stainless steel, with a sectional area of 5 mm² or less, a tapered slope circumferentially in contact with the body part and formed at a predetermined inclination angle θ with respect to an axis of the body part, and a tapered tip circumferentially in contact with the tapered slope and formed at a predetermined inclination angle ϕ with respect to the axis of the body part, **characterized in that** the inclination angle θ of the tapered slope is larger than the inclination angle ϕ of the tapered tip, and the tapered tip is formed of a stainless crystal structure having a crystal grain size of 1 µm or less.

3. A method for manufacturing a non-magnetic fine stainless steel processed product, comprising a tapered slope forming step of forming a tapered slope by cutting diagonally stainless steel having a fine shape that is either a polygonal or cylindrical stainless steel, or polygonal or cylindrical hollow stainless steel, with a sectional area of 5 mm² or less, a tapered tip crush-forming step of forming a tapered tip formed of a stainless crystal structure having a crystal grain size of 1 µm or less by warm-crushing a tip end part of the tapered slope, and a tapered tip grinding step of grinding the warm-crushed tapered tip.

4. The method for manufacturing a non-magnetic fine stainless steel processed product according to claim 3, **characterized in that** the tapered tip crush-forming step of forming the tapered tip composed of the stainless crystal structure having the crystal grain size of 1 µm or less is warm crushing that is performed by keeping the tapered slope at a plastic deformation temperature of 200°C or more and 300°C or less with a temperature variation of 30°C or less.
